# EUROPÄISCHE PATENTANMELDUNG

(11) **EP 3 159 015 A1**
(43) Veröffentlichungstag der Anmeldung: **26.04.2017**
(21) Anmeldenummer: 16193233.0
(22) Anmeldetag: 11.10.2016
(51) Int. Cl.: A61L 2/18, B08B 3/04, C11D 1/12, C11D 3/48

(54) **VERFAHREN ZUR REINIGUNG UND DESINFEKTION VON TRINKWASSERBEHÄLTERN**

(30) Priorität: 21.10.2015 DE 102015117955
(71) Anmelder: Witty GmbH & Co. KG, 86424 Dineklscherben (DE)
(72) Erfinder: Braun, Brigitte, 86368 Gersthofen (DE); Golz, Dr. Gregor, 86157 Augsburg (DE)
(74) Vertreter: Charrier Rapp & Liebau

(57) **Zusammenfassung**

Die Erfindung betrifft ein Verfahren zur Reinigung und Desinfektion von Trinkwasserbehältem, wobei ein Reinigungs- und Desinfektionsmittel, umfassend ein Gemisch von C₁-C₆-Alkansulfonsäure und Wasserstoffperoxid auf die zu behandelnden Innenflächen des Trinkwasserbehälters aufgetragen und nach einer Einwirkungszeit mit Wasser abgespült wird, wobei bezogen auf das Gewicht mehr Alkansulfonsäure als Wasserstoffperoxid verwendet wird.

## Beschreibung

Die Erfindung betrifft ein Verfahren zur Reinigung und Desinfektion von Trinkwasserbehältern.

Die Trinkwasserverordnung (TrinkwV) fordert im Zusammenhang mit anderen Regulierungen (DIN EN 805:2000; DVGW W 347 (A); DIN 1045; DIN EN 206-1; DIN EN 12390-8; LMBGJ; DVGW W 291 (A); Biozid-VO 528/2012, EN 14885) eine regelmäßige Reinigung und Desinfektion von Trinkwasserbehältern der öffentlichen Trinkwasserversorgung, damit keine gefährdenden Stoffe in das Trinkwasser gelangen und/oder deren Höchstmengen nicht überschritten werden.

Eisen und Mangan können in sauerstoffarmem Wasser gelöst sein. Sie sind im Trinkwasser mineralischen Ursprungs. Wird dieses Wasser belüftet oder mit Sauerstoff-reichem Wasser gemischt, fallen Eisenoxidhydrate und Mangandioxid aus. Auch in ausreichend belüftetem Wasser sind noch geringste Mengen Eisen und Mangan enthalten, die im Dauerbetrieb zu entsprechenden Ablagerungen in Trinkwasserbehältern führen und die benetzten Flächen dunkel färben.

Erhärtete Krusten dieser Ablagerungen sind insbesondere im Bereich der Wasserwechselzone an den Wänden und Stützen der Trinkwasserbehälter schwierig zu entfernen. Darüber hinaus können sich Eisen und Mangan als festhaftende, oxidische Ablagerungen darstellen. Sollen diese entfernt werden, können chemische Reinigungsmittel nützlich sein.

Die GB826172-A offenbart ein Verfahren zum Entfernen von Kalkablagerungen und Rost von den Innenflächen von Rohrleitungen, wobei das verwendete wässrige Mittel Wasserstoffperoxid und Phosphorsäure enthält. Die US 4806169-B betrifft ein Verfahren zur Entfernung von festen Ablagerungen aus Wasserinstallationen. Dabei wird zur Entfernung von Eisen-, Calcium- und Mangan-haltigen Inkrustationen Mineralsäure und H₂O₂ auf die mit Inkrustationen verunreinigten Einrichtungen appliziert.

In bekannten Verfahren zur Reinigung von Trinkwasserbehältern wird zur Beseitigung vor allem der Eisen- und/oder Mangan-Beläge Schwefelsäure oder Phosphorsäure in hoher Konzentration durch Aufsprühen verwendet. Um die Beläge besser zu lösen, wird gemeinsam mit der Schwefelsäure oder Phosphorsäure Wasserstoffperoxid aufgetragen. Dabei müssen Säure und Wasserstoffperoxid wegen der raschen Zersetzung des Letzteren gesondert gelagert werden und werden kurz vor dem Auftragen vereint. Bevorzugt werden sie in situ z.B. in einer Sprühlanze während des Auftragungsvorgangs vermischt. Eine Lagerung der beiden Stoffe verbietet sich z.B. auch wegen der Bildung von Caroscher Säure, die mit organischen Stoffen hochexplosiv reagieren kann. Anschließend erfolgt üblicherweise das Auftragen eines Desinfektionsmittels, z.B. eines Oxidationsmittels für organische Stoffe, wie chlorhaltige Stoffe oder Wasserstoffperoxid.

Aufgabe der vorliegenden Erfindung ist die Bereitstellung eines vereinfachten Verfahrens zur Reinigung und Desinfektion von Trinkwasserbehältern, das die Gründlichkeit der bisherigen Verfahren erzielt oder übertrifft, welches aber keine gesonderte Aufbewahrung der Bestandteile des Reinigungsmittels in getrennten Behältern erfordert und welches mit einer üblichen Ausrüstung, z.B. einer Sprühlanze (und bevorzugt ohne Mischkopf) auf die zu reinigenden und desinfizierenden Oberflächen aufgebracht werden kann.

Diese Aufgabe wird erfindungsgemäß durch ein einstufiges Verfahren zur Reinigung von Trinkwasserbehältern gelöst, in dem ein flüssiges Reinigungs- und Desinfektionsmittel, welches ein Gemisch von C₁-C₆-Alkansulfonsäure und Wasserstoffperoxid enthält, auf die Innenflächen des zu behandelnden Trinkwasserbehälters aufgetragen und nach einer Einwirkungszeit mit Wasser abgespült wird, wobei das Reinigungsmittel bezogen auf das Gewicht mehr Alkansulfonsäure als Wasserstoffperoxid enthält. Soweit im Folgenden vereinfacht von Reinigungsmittel gesprochen wird, ist das erfindungsgemäß verwendete Reinigungs- und Desinfektionsmittel gemeint, welches eine C₁-C₆-Alkansulfonsäure und Wasserstoffperoxid enthält und als fertiges Gemisch vorliegt und in dem mehr Alkansulfonsäure als Wasserstoffperoxid enthalten ist.

Die im erfindungsgemäßen Reinigungsmittel enthaltene Alkansulfonsäure kann oberflächliche Verschmutzungen und Verkrustungen aus Magnesium-, Calcium-, Mangan- und/oder Eisen-haltigen Stoffen beseitigen. Das erfindungsgemäße Reinigungsmittel wirkt dabei gleichzeitig aufgrund der Anwesenheit des Wasserstoffperoxids desinfizierend. Das Wasserstoffperoxid unterstützt weiterhin die Entfernung der genannten Verschmutzungen und Verkrustungen von den zu behandelnden Oberflächen und verbessert somit die Reinigungsleistung. Dadurch können Oberflächen durch Applikation des erfindungsgemäßen Reinigungsmittels in einem einzigen Applikationsschritt sehr effizient gleichzeitig gereinigt und desinfiziert werden. Die Applikation des erfindungsgemäßen Reinigungsmittels auf eine zu behandelnde Oberfläche kann dabei auf einfache und zeitsparende Weise erfolgen, da das Reinigungsmittel als gebrauchsfertige Lösung vorliegt und bspw. mit einem Sprühschlauch oder einer Sprühlanze auf die zu behandelnde Oberfläche aufgebracht werden kann. Eine Vermischung von Komponenten des Reinigungsmittels vor dessen Applikation ist nicht erforderlich.

Vorteilhafte Weiterbildungen und Ausgestaltungen der Erfindung sind in den Unteransprüchen angegeben.

Üblicherweise werden die bei großen Trinkwasserbehältern nach längerer Zeit auftretenden Verkrustungen mit starker Mineralsäure gelöst und der befreite Untergrund anschließend mit einem Oxidationsmittel keimfrei gemacht. Bei den Oxidationsmitteln handelt es sich unter anderem um halogenhaltige oder Wasserstoffperoxid-haltige Mittel. Es ist nicht möglich, Oxidationsmittel und starke Säuren gemeinsam als ein gebrauchsfertiges Behandlungsmittel zu konfektionieren und in einem Aufbewahrungsbehälter zu lagern. Halogenhaltige Oxidationsmittel sind in der Regel stark alkalische Hypohalogenite (z.B. NaOCl), die mit einer starken Säure nicht vereinbar sind. Der Einsatz von Salzsäure (HCl) verbietet sich, wenn der Trinkwasserbehälter Wände aus Edelstahl aufweist. Daher wird als starke Mineralsäure zumeist Schwefelsäure (H₂SO₄) oder Phosphorsäure (H₃PO₄) eingesetzt. Eine Mischung von Schwefelsäure oder Phosphorsäure mit Wasserstoffperoxid ist wenig stabil und z.B. durch die Bildung von Caroscher Säure (H₂SO₅) zudem gefährlich. Aus diesem Grunde müssen beide Komponenten separat in getrennten Aufbewahrungsbehältern gelagert werden und werden erst vor Ort unmittelbar vor dem Auftragen auf die zu reinigenden Oberflächen des Trinkwasserbehälters vermischt, bspw. in einer Mischdüse einer Sprühlanze.

Trinkwasserbehälter, die als Trinkwasser-Reservoir dienen, können viele m³ Wasser enthalten, sie sind in der Regel begehbar und können aus den verschiedensten Materialien, wie geflieste Flächen, mit Kunststoff ausgekleidete Flächen, Edelstahlbleche oder Beton bestehen. Es hat sich allerdings gezeigt, dass insbesondere der am häufigsten für diese Zwecke verwendete Beton mit einem Schutzanstrich versehen werden muss, weil Wasser z.B. in Kombination mit Kohlendioxid den Beton auf Dauer angreift, durch hydrolytische Korrosion auslaugt und dabei porös macht, u.U. bis hin zu der Bewehrung. Aus diesem Grunde werden Anstriche, vor allem aus mineralischem Zementmörtel, auf das Innere eines aus Beton gefertigten Trinkwasserbehälters aufgetragen. Diese Zemente sind Spezialzemente mit einem besonders hohen Calciumoxidanteil. Sie enthalten hauptsächlich Zement, Titandioxid, Microsilica und Calciumcarbonat und werden in Schichten mit einer Dicke von 5 bis 30 mm aufgetragen.

Überraschenderweise wurde nun gefunden, dass die Verwendung eines Reinigungs- und Desinfektionsmittels, welches Methansulfonsäure und Wasserstoffperoxid und gegebenenfalls weitere Komponenten enthält, bei der Reinigung und Desinfektion von Trinkwasserbehältern gleiche und sogar bessere Leistungen erzielt als der derzeit übliche Einsatz der bekannten Zweikomponenten-Mittel aus bspw. Schwefelsäure oder Phosphorsäure und Wasserstoffperoxid, wenn das Reinigungsmittel bezogen auf das Gewicht mehr Alkansulfonsäure als Wasserstoffperoxid enthält. Außerdem ist zur Bewältigung der Aufgabe einen Trinkwasserbehälter zu säubern und zu desinfizieren nur ein einziger Vorgang (einstufiges Verfahren) erforderlich, im Gegensatz zum herkömmlichen Verfahren bei dem zuerst Eisen- und Mangan-Verkrustungen gelöst werden müssen und danach im Anschluss mit einem Biozid die Desinfektion erfolgt. Der Vorteil des erfindungsgemäß eingesetzten Reinigungsmittels gegenüber den bekannten zweikomponentigen Reinigungsmmitteln liegt insbesondere darin, dass das erfindungsgemäß eingesetzte Reinigungsmittel als gebrauchsfertiges Konzentrat oder als gebrauchsfertige wässrige Lösung bereit gestellt werden kann, welches in einem Aufbewahrungsbehälter abgefüllt und über einen längeren Zeitraum von bevorzugt mindestens einem Jahr gelagert werden kann, ohne bei der Lagerung einen wesentlichen Verlust an Wirksamkeit zu erleiden. Dadurch entfällt die Notwendigkeit einer komplizierten Auftragungstechnik, z.B. mit einer Sprühlanze mit integrierter Mischvorrichtung, in der die Komponenten des Reinigungsmittels noch vermischt werden müssen. Auch die Abfüllung sowie die Lagerung und der Transport des Reinigungsmittels vereinfachen sich erheblich, da keine getrennten Aufbewahrungsbehälter für die Komponenten des Reinigungsmittels bereit gestellt werden müssen. Außerdem ist Methansulfonsäure weniger gefährlich als Schwefelsäure, insbesondere bei der Herstellung von an den jeweiligen Anwendungsfall und den Verschmutzungsgrad des zu reinigenden Trinkwasserbehälters angepassten Verdünnungen. Ein Nachteil bekannter Problemlösungen besteht darin, dass man bei der Verwendung eines Gemisches aus Mineralsäure und Wasserstoffperoxid nicht verbrauchten Ansatz speziell entsorgen muss. Durch die Lagerfähigkeit des in der Erfindung verwendeten Reinigungs- und Desinfektionsmittels kann man nicht verwendetes Mittel aufbewahren und später oder an einem anderen Ort verbrauchen.

Das erfindungsgemäß verwendete Reinigungsmittel enthält neben einer C₁-C₆-Alkansulfonsäure noch Wasserstoffperoxid (H₂O₂). In der Regel wird handelsübliches technisches Wasserstoffperoxid mit einer Konzentration zwischen 35 und 50% zum Anmischen des erfindungsgemäßen Reinigungsmittels eingesetzt. Wasserstoffperoxid wird bei unter 15°C gelagert. In der vorliegenden Erfindung wird Wasserstoffperoxid bevorzugt in einer Menge von 4 bis 30 Gew.%, vorzugsweise 6 bis 20 Gew.%, insbesondere 7 bis 10 Gew.% eingesetzt. Bevorzugt sind Konzentrationen des Wasserstoffperoxid in dem Reinigungsmittel gleich oder unter 8 Gew.-%, weil das Gemisch des Reinigungsmittels dann als nicht brandfördernd eingestuft werden kann, was bei der Lagerung von Vorteil ist.

Als Säurekomponente wird eine C₁-C₆-Alkansulfonsäure eingesetzt. Bevorzugte C₁-C₆-Alkansulfonsäuren sind Ethansulfonsäure und Methansulfonsäure. Letztere ist aufgrund ihrer Eigenschaften bevorzugt, insbesondere weil damit hergestellte Mittel geruchsneutral sind. Methansulfonsäure ist eine starke Säure, die mit Wasser in jedem Verhältnis mischbar ist und selbst als Lösemittel dienen kann, da sie ab etwa Raumtemperatur flüssig ist. Sie ist unbegrenzt mit Wasser oder Ethanol mischbar. Der Dampfdruck ist ähnlich niedrig wie der von Schwefelsäure. Methansulfonsäure wirkt auch in hohen Konzentrationen nicht oxidierend. Handelsüblich ist eine Konzentration von etwa 70%, z.B. Lutropur® MSA von BASF. Sie ist zudem nach der OECD Richtlinie 301 A biologisch leicht abbaubar, wobei Kohlendioxid und Sulfat als Abbauprodukte entstehen. In der vorliegenden Erfindung wird Methansulfonsäure als aktiver Stoff bevorzugt in einer Menge von 20 bis 50 Gew.%, besonders bevorzugt von 23 bis 45 Gew.%, insbesondere von 25 bis 42 Gew.% eingesetzt. Ganz besonders bevorzugt ist eine Menge von 35 bis 40 Gew.%.

Das erfindungsgemäß verwendete Reinigungsmittel enthält gegebenenfalls Additive als Hilfskomponenten, wie z.B. Tenside, Wasserstoffperoxid-Stabilisatoren, Verdicker, Natriumalkylsulfonate, Entschäumer, hydrotropen Mittel oder Gemische davon.

Der Mischung aus Wasserstoffperoxid und Methansulfonsäure des erfindungsgemäßen Reinigungsmittels kann zweckmäßig Hydroxyethylidendiphosphonsäure (Etidronsäure bzw. HEDP) zugesetzt werden. Diese hat chelat-bildende Eigenschaften für Calcium, Eisen und andere Schwermetallionen. Außerdem besitzt sie korrosionshemmende Eigenschaften für unlegierten Stahl. Eine für die vorliegende Erfindung wichtige Eigenschaft ist jedoch die Stabilisierung von Wasserstoffperoxid. Durch den Einsatz von Etidronsäure kann somit die Lagerfähigkeit deutlich verlängert werden. Das Produkt wird in der Regel in einer Konzentration von 60% geliefert. Etidronsäure wird vorzugsweise in einer Menge von 0,3 bis 2,0 Gew.%, vorzugsweise 0,4 bis 1 Gew.%, insbesondere 0,5 bis 0,7 Gew.% verwendet.

Das Verhältnis von Methansulfonsäure zu Wasserstoffperoxid als aktiver Bestandteil des Reinigungsmittel-Konzentrats ist dergestalt, dass bezogen auf die Masse mehr Methansulfonsäure vorliegt als Wasserstoffperoxid. Es hat sich gezeigt, dass ein sehr guter Reinigungseffekt erzielt wird, wenn ein Überschuss an Methansulfonsäure vorliegt. Insbesondere liegt das Gewichts-Verhältnis von Methansulfonsäure zu Wasserstoffperoxid im Bereich von 10:1 bis 1,1:1, vorzugsweise 8:1 bis 1,1:1. Vor allem hat sich ein Verhältnis von 7:1 bis 5:1 und bevorzugt um 6:1 als besonders geeignet erwiesen.

Zu der in der vorliegenden Erfindung verwendeten Mischung aus Wasserstoffperoxid und Methansulfonsäure kann weiterhin ein Tensid gegeben werden. Bevorzugt sind nichtionische Tenside, bevorzugter Fettalkoholalkoxylate, insbesondere Fettalkoholethoxylate oder Fettalkoholethoxylatpropoxylate, wie jene aus der Plurafac-Reihe, z.B. Plurafac® LF231, Plurafac® LF303, Biodac®-232 und/oder Hoesch® FA64LF. Die Fettalkoholalkoxylate weisen die Formel

R-O-(R'-O)ₙ-H

auf, worin R einen C₄-C₁₈, vorzugsweise C₈-C₁₆-Alkyl-Rest darstellt, R' Ethylen, Methylethylen, Propylen und/oder Isopropylen bedeutet und n ein Wert von 2 bis 30, vorzugsweise 4 bis 20, insbesondere 6 bis 16 ist.

Fettalkoholethoxylate können zur Verbesserung der Löslichkeiten verwendet werden. Sie sind umwelttechnisch unbedenklich. In der vorliegenden Erfindung werden Fettalkoholalkoxylate, bspw. in einer Menge von bis zu 0,1 Gew.%, vorzugsweise von 0,05 Gew.% bis 0,3 Gew.%, und insbesondere von 0,15 Gew.% bis 0,25 Gew.-% verwendet. Vorzugweise wird kein Tensid eingesetzt.

Das Reinigungsmittel kann zudem einen Verdicker enthalten, der die Ablaufzeit des Mittels, insbesondere an vertikalen oder schrägen Wänden eines Trinkwasserbehälters und damit die Einwirkzeit erhöht. Hierzu eignen sich bspw. Verdickungsmittel, die in stark sauren Lösungen stabil sind. Diese können vom nichtionischen Polyurethan-Typ in Kombination mit Fettalkoholalkoxylat-Typen sein, wobei der Fettalkohol verzweigt ist. Handelsüblich ist z.B. Rheosolve® 450. Nützlich sind auch hydrophob modifizierte ethoxylierte Urethan-Copolymere und/oder hydrophob modifizierte Hydroxyethyl-Celluloseether. Des Weiteren können Alkyldimethylaminoxide oder Alkyl-(2-hydroxyethyl)-aminoxide genannt werden, wie Kokos-bis-(2-hydroxyethyl)-aminoxid oder Talg-bis-(2-hydroxyethyl)-aminoxid. Handelsübliche Stoffe sind GENAMINOX^{®} CSL, MACKAMINE^{®} CS oder AROMOX^{®} MCD-W.

Des Weiteren kann das erfindungsgemäß verwendete Reinigungsmittel auch Natriumalkylsulfonate, wie Natrium-C₁₄-C₁₇-sec-alkylsulfonate enthalten. Diese können in einer Menge, wie die vorstehend genannten Fettalkoholalkoxylat-Tenside eingesetzt werden.

Es können auch Entschäumer eingesetzt werden, um den Auftragungsvorgang zu verbessern. Hierfür eignen sich beispielsweise Ethoxy-propoxylierte Fettalkohole. Sie können in Mengen äquivalent zu vorstehend genannten Tensiden eingesetzt werden. Das erfindungsgemäße Reinigungsmittel kann damit besonders gut aufgesprüht werden, bspw. mit einer Sprühlanze.

Schließlich kann das erfindungsgemäß verwendete Reinigungsmittel auch ein hydrotropes Mittel enthalten, z.B. Cumol- und/oder Xylolsulfonate. Diese können, falls verwendet, in einer Konzentration bis zu 20%, vorzugsweise bis zu 10% eingesetzt werden.

Das erfindungsgemäß verwendete Reinigungsmittel kann vorzugsweise in einer Mischanlage hergestellt werden, indem eine berechnete Menge Wasser (vorzugsweise ein deionisiertes Wasser) vorgelegt wird und die zugehörige Menge Alkansulfonsäure, z.B. Methansulfonsäure, allmählich unter Einsatz eines Rührwerks zumischt wird. Der Vorgang ist exotherm. Nach Abkühlen werden weitere Additive, wie Tenside und insbesondere auch das Wasserstoffperoxid stabilisierende HEDP (Etidronsäure) zugegeben. Nach Abkühlen auf etwa Raumtemperatur oder darunter, z.B. 15°C, wird die berechnete Menge Wasserstoffperoxid zugemischt. Das fertige Reinigungsmittelgemisch wird dann zweckmäßig in Kunststoffbehälter (bspw. aus Polyethylen oder Polypropylen) in Mengen bis zu mehreren Litern abgefüllt und ist ungeöffnet bevorzugt mindestens ein Jahr bei Raumtemperatur ohne Einbuße der Reinigungskraft lagerfähig.

Bei der Herstellung des Reinigungsmittels, insbesondere bei der Auswahl der Komponenten, ist darauf zu achten, dass organische Additive, wie Tenside, als Hilfsstoffe nur im entsprechend passenden Einsatzfall anzuwenden sind. Generell sollte das Konzentrat so wenige wie möglich chemische Bestandteile enthalten, da diese anschließend restlos fortgespült werden müssen. Tenside sind aufgrund ihrer Amphiphilie beim Ablösen von z.B. organischen Kohlenstoff enthaltenden Belägen hilfreich, sind aber als Rückstand hartnäckiger als Wasserstoffperoxid und Methansulfonsäure und erfordern daher einen höheren Spülaufwand mit Trinkwasser, bis die vorgeschriebenen Grenzwerte für organischen Kohlenstoff (TOC und DOC) erreicht sind.

Das Reinigungsmittel wird bevorzugt als unverdünntes Konzentrat auf die zu reinigende Oberfläche eines Trinkwasserbehälters appliziert. Als Konzentrat wird hierin ein Reinigungsmittel bezeichnet, das wenigstens 30 Gew.-% Alkansulfonsäure (z.B. Methansulfonsäure) enthält. Es kann jedoch auch das zunächst in einem Aufbewahrungsbehälter als unverdünntes Konzentrat vorliegende Reinigungsmittel vor Ort mit Wasser verdünnt und dann als wässrige Lösung manuell auf die zu reinigende Oberfläche aufgetragen werden. Geeignete Verdünnungen des Konzentrats richten sich nach dem Zustand der zu behandelnden Flächen, insbesondere dem Verschmutzungsgrad und der Art der Verschmutzungen. Je nach Verschmutzungsgrad wird das in einem Aufbewahrungsbehälter abgefüllte unverdünnte Konzentrat bspw. mit Wasser im Verhältnis von 1:1 bis 1:10 verdünnt. Bei geringeren Verschmutzungen können auch Verdünnungen von 1:100 eingesetzt werden. Besonders bei stark verkrusteten Verschmutzungen erweist es sich jedoch als zweckmäßig, das Reinigungsmittel als unverdünntes Konzentrat auf die zu reinigende Oberfläche zu applizieren.

Zum Auftragen des erfindungsgemäß verwendeten Reinigungsmittels eignen sich unter anderem säurebeständige Sprühpistolen oder Sprühlanzen. Dies erhöht den Applikationsbereich und die Auftragseffizienz und sorgt überdies auch für einen gleichmäßigen Auftrag und einen größeren Abstand zwischen dem regelmäßig mit einer Schutzkleidung ausgestatteten Anwender und dem Austrittsort des auch im verdünnten Zustand noch aggressiven Mittels.

Geeignete Sprühlanzen zum Applizieren des erfindungsgemäßen Reinigungsmittels auf eine zu reinigende und zu desinfizierende Oberfläche eines Trinkwasserbehälters können bis zu 4 m Länge aufweisen, in besonderen Ausführungen sogar bis zu 14 m. Der Auftrag erfolgt zweckmäßig mit einem Druck im Bereich von bis zu 10 bar bei einer Auftragsmenge von 1 bis 20 l/min, vorzugsweise 2 bis 10 l/min, insbesondere 3 bis 5 l/min. Als besonders geeignete Auftragsvorrichtung erweist sich bspw. das Witty-RUDY^{®} Reinigungssystem für Trinkwasserbehälter.

Das Reinigungsmittel kann jedoch auch auf andere Weise aufgetragen werden. Dazu eignen sich bspw. Pinsel, Bürsten, Schwämme und Auftragsrollen sowie Spritzschläuche mit oder ohne Düse oder nach dem Injektorprinzip, oder andere Auftragungsmittel oder -geräte.

Nach Einsprühen der zu reinigenden Oberflächen werden - je nach Verschmutzungs- und Verkrustungsgrad - Einwirkzeiten von bis zu 30 Minuten oder zweckmäßig bis zu 15 Minuten, vorzugsweise bis zu 10 Minuten oder 5 Minuten, unter Umständen nur bis zu 1 Minute ausreichen, um die Belagstellen zu beseitigen. Für hartnäckige Verschmutzungen und Verkrustungen hat sich der Zusatz eines Verdickungsmittels bewährt, um die Ablaufzeit des Reinigungsmittels und damit dessen Einwirkzeit zu erhöhen. Insbesondere an den vertikalen oder nur leicht geneigten Wandungen eines Trinkwasserbehälters kann so eine höhere Verweilzeit (Einwirkzeit) des Reinigungsmittels und dadurch ein zufriedenstellendes Reinigungsergebnis erzielt werden. Durch das erfindungsgemäße Verfahren können außerdem Verkrustungen viel leichter in einem Arbeitsschritt entfernt und die Fläche gleichzeitig desinfiziert werden, während bei herkömmlicher Vorgehensweise (bspw. Auftragen von Mineralsäure im Gemisch mit Wasserstoffperoxid in einem ersten Schritt und anschließende Desinfektion in einem zweiten Schritt), dem Auftragungsvorgang eine arbeitsintensive mechanische Lockerung mit Abtragungsgeräten vorangehen muss.

Nach dem Einwirken des Reinigungsmittels wird der gereinigte Trinkwasserbehälter gründlich mit Wasser gespült, bis eine Probe keine Rückstände des eingesetzten Reinigungsmittels mehr anzeigt. Es ist erforderlich, das vollständige Abspülen des Reinigungsmittels zu kontrollieren, da im Trinkwasserbehälter verbleibende Reinigungsmittelreste keine nachteiligen Wirkungen auf die Qualität des Trinkwassers aufweisen dürfen. Hierfür wird eine Probe auf Rückstände des eingesetzten Reinigungsmittels untersucht. Um die toxikologische und trinkwasserhygienische Eignung des Reinigungsmittels zur Reinigung von Trinkwasserbehälters unter Berücksichtigung der Anforderungen des Lebensmittel- und Bedarfsgegenständegesetzes (LMG3) und die Trinkwasserverordnung (TrinkwV) zu beurteilen, werden standardisierte Tests an Testplatten auf vorgegebenen Zielmaterialien durchgeführt. Für diese Untersuchungen werden Testplatten mit Kantenlängen von 8 x 15 cm aus Zementmörtel, Edelstahl und Plexiglas eingesetzt. Sie werden von einer Prüfstelle gestellt und gemäß dem vorgeschriebenen Behandlungsvorgang mit dem Reinigungsmittel behandelt (insbesondere 1 Stunde dem Reinigungsmittel der jeweils vom Hersteller angegebenen, höchsten Anwendungskonzentration ausgesetzt) und anschließend eine vorgeschriebene Zeit, bspw. 24 Stunden, in dem Testwasser gelagert. Es wird festgestellt, ob Reste des Reinigungsmittels an das Testwasser abgegeben werden, eine Vermehrung von Mikroorganismen auftritt oder Wirkungen auf die behandelten Werkstoffe bzw. auf das Trinkwasser ausgehen.

Vor Ort können zur Kontrolle bezüglich des Wasserstoffperoxids Teststreifen verwendet werden. Die Alkansulfonsäure, bspw. Methansulfonsäure, ist über den pH-Wert messbar, sobald dieser den Wert des Spülwassers erreicht hat. Außerdem erfolgen gegebenenfalls mikrobiologische Untersuchungen. Genauere Laboruntersuchungen können gemäß den Pflichtprüfungen bei dem Test von Reinigungsmitteln erfolgen. Dabei wird der organisch gebundene Kohlenstoff (TOC, Methode H3 der deutschen Einheitsverfahren (DEV)), an gelöstem organischen Kohlenstoff (DOC) und der Grad an vermehrungsfähigen Keimen (koloniebildende Einheiten, KBE), Methode K5 der DEV, beurteilt.

Das erfindungsgemäß verwendete Reinigungsmittel besteht aus rückstandsfrei abspülbaren Komponenten. Die Entsorgung der Einsatzchemikalien kann sehr umweltverträglich zu den bestehenden Regulierungen erfolgen. Bedenkliche Chemikalien, die auf dem Fachgebiet üblich sind, wie chlorhaltige Oxidationsmittel (Hypochlorit oder Chlordioxid) oder Perborat werden nicht verwendet. Dasselbe gilt für die ebenfalls als bedenklich befundene Phosphorsäure.

Die Erfindung wird nachfolgend anhand eines Ausführungsbeispiels erläutert. Die Erfindung ist allerdings nicht auf den Umfang des Beispiels begrenzt, vielmehr wird der Umfang durch die Ansprüche definiert. Alle Prozent- und Teilangaben in dem Beispiel und der gesamten Beschreibung sind auf das Gewicht bezogen, sofern nichts anderes ausgewiesen worden ist.

### Beispiel

Ein Reinigungsmittel-Konzentrat gemäß der Erfindung wird in einer Mischanlage hergestellt. Dazu wird Wasser (vorzugsweise ein deionisiertes Wasser) vorgelegt und mit der angegebenen Menge an Methansulfonsäurelösung unter Wärmeentwicklung vermischt. Nach Abkühlen auf eine gemäßigte Temperatur werden die weiteren Additive, insbesondere HEDP (Etidronsäure), außer Wasserstoffperoxid, zugemischt. Nach Abkühlen auf Raumtemperatur oder darunter wird dann die erforderliche Menge Wasserstoffperoxid zugegeben. Eine bevorzugte Zusammensetzung ist in der nachstehenden Tabelle 1 angegeben:

**Tabelle 1**

| **Inhaltsstoff** | **Handelsname** | **Gehalt aktiver Bestandteil im Ausgangsstoff** % | **CAS-Nummer** | **Aktiver Bestandteil in Formulierung** % |
|---|---|---|---|---|
| Wasserstoffperoxid | | 49,5 | 7722-84-1 | 7,950 |
| Hydroxyethylidendiphosphonsäure (Etidronsäure) | | 60 | 2809-21-4 | 0,609 |
| Fettalkoholethoxylate | Plurafac^{®} LF231, Plurafac^{®} LF303, Biodac^{®}-232, Hoesch^{®} FA64LF | 100 | polymer | 0-1,0 bzw. 0-0,2 |
| Methansulfonsäure | Lutropur^{®} MSA | 70 | 75-75-2 | 38,5 |
| Wasser deionisiert | | 100 | | auf 100 % |

Das so hergestellte Reinigungsmittel-Konzentrat wird in Kunststoffbehälter aus Polyethylen oder Polypropylen bspw. in einer Menge von 10 Litern abgefüllt und ist ungeöffnet mindestens ein Jahr ohne Einbuße der Reinigungskraft bei Raumtemperatur lagerfähig.

## Patentansprüche

1. Verfahren zur Reinigung und Desinfektion von Trinkwasserbehältern, wobei ein flüssiges Reinigungs- und Desinfektionsmittel, welches ein Gemisch von C₁-C₆-Alkansulfonsäure und Wasserstoffperoxid enthält, auf die Innenflächen des zu behandelnden Trinkwasserbehälters aufgetragen und nach einer Einwirkungszeit mit Wasser abgespült wird, wobei das Reinigungs- und Desinfektionsmittelbezogen auf das Gewicht mehr Alkansulfonsäure als Wasserstoffperoxid enthält.

2. Verfahren nach Anspruch 1, wobei das Reinigungs- und Desinfektionsmittel als gebrauchsfertiges und lagerfähiges Gemisch in konzentrierter Form oder als wässrige Lösung vorliegt und wobei eine konzentrierte Form so definiert ist, dass sie mindestens 30 Gew.-% Alkansulfonsäure enthält.

3. Verfahren nach Anspruch 1 oder 2, wobei das Gewichtsverhältnis von Alkansulfonsäure zu Wasserstoffperoxid 10:1 bis 1,1:1, vorzugsweise 7:1 bis 5:1 und besonders bevorzugt 6:1 beträgt.

4. Verfahren nach einem der vorangehenden Ansprüche, wobei die Innenflächen des zu behandelnden Trinkwasserbehälters vertikal stehen oder höchstens eine Neigung von +/- 25° zur Vertikalen aufweisen.

5. Verfahren nach einem der vorangehenden Ansprüche, wobei es sich bei der Alkansulfonsäure um Methansulfonsäure handelt.

6. Verfahren nach einem der vorangehenden Ansprüche, wobei das Reinigungs- und Desinfektionsmittel zusätzlich noch Additive enthält, welche aus einem oder mehreren Tensiden, Wasserstoffperoxid-Stabilisatoren, Verdickern, Natriumalkylsulfonaten, Entschäumern, hydrotropen Mitteln oder Gemischen davon ausgewählt sind.

7. Verfahren nach einem der vorangehenden Ansprüche, wobei das Reinigungs- und Desinfektionsmittel gebrauchsfertig als Konzentrat oder als wässrige Lösung in Vorratsbehältern abgefüllt und nach Abfüllung für mindestens 1 Jahr gebrauchsfähig lagerbar ist.

8. Verfahren nach einem der vorangehenden Ansprüche, wobei die zu beseitigenden Verschmutzungen Magnesium-, Calcium-, Mangan- und/oder Eisen-haltige Verkrustungen sind.

9. Verfahren nach einem der vorangehenden Ansprüche, wobei der Gewichtsanteil des Wasserstoffperoxid kleiner als 8% ist.
